# EUROPÄISCHE PATENTSCHRIFT

(11) **EP 2 375 993 B1**
(45) Veröffentlichungstag und Bekanntmachung des Hinweises auf die Patenterteilung: **22.11.2017**
(21) Anmeldenummer: 09775085.5
(22) Anmeldetag: 17.12.2009
(51) Int. Cl.: A61B 17/00, B05B 7/08, B05B 11/06, B05C 17/005, F16K 31/60

(54) **AUSTRAGVORRICHTUNG MIT DRUCKGASUNTERSTÜTZUNG**
DISCHARGE APPARATUS HAVING COMPRESSED GAS SUPPORT
DISPOSITIF DE DISTRIBUTION ASSISTÉ PAR DU GAZ COMPRIMÉ

(30) Priorität: 13.01.2009 CH 45092009
(43) Veröffentlichungstag der Anmeldung: 19.10.2011
(73) Patentinhaber: Medmix Systems AG, 6343 Rotkreuz (CH)
(72) Erfinder: GRETER, Andy, 6648 Minusio (CH); BREM, William, 5630 Muri (CH)
(74) Vertreter: Detken, Andreas
(86) Internationale Anmeldenummer: PCT/CH2009/000400
(87) Internationale Veröffentlichungsnummer: WO 2010/081244

(56) Entgegenhaltungen:
- WO-A-95/31138
- US-A- 4 645 496
- US-A- 5 582 596

## Beschreibung

### TECHNISCHES GEBIET

Die vorliegende Erfindung betrifft eine Austragvorrichtung mit Druckgasunterstützung zum Aufsprühen eines Produkts auf eine Anwendungsfläche, insbesondere zum Aufsprühen von Zweikomponentenprodukten auf eine Behandlungsfläche im Rahmen einer medizinischen Anwendung. Ferner betrifft die Erfindung ein Verfahren zum Austragen eines Produkts aus einer solchen Austragvorrichtung.

### STAND DER TECHNIK

Bei verschiedenen Anwendungen ist es erforderlich, ein meist in fluider Form vorliegendes Produkt in einem Behälter, wie z. B. einer Spritze aufzubewahren und bei der Anwendung durch Versprühen auf eine Oberfläche aufzutragen. Beispielsweise werden Gewebeklebstoffe, wie z. B. Fibrin oder Thrombin, in Form von getrennten Komponenten in einer Doppelspritze gelagert und können mit Hilfe einer Austragvorrichtung, die eine Druckgasunterstützung aufweist auf eine Gewebeoberfläche gesprüht werden. Die beiden Klebstoffkomponenten werden dabei ausserhalb einer Sprühdüse der Austragvorrichtung in dem Sprühnebel, der durch die Druckgaseinrichtung erzeugt wird, miteinander vermischt, um ein Verkleben der Sprühdüse zu verhindern. Das Aufsprühen auf die Gewebeoberfläche kann dabei unterbrochen werden, ohne dass die Sprühdüse verklebt. Solche Klebstoffe werden z. B. für die Wundbehandlung oder das Stoppen von Blutungen eingesetzt.

Eine Austragvorrichtung mit Druckgasunterstützung ist z. B. aus der US 2002/0165483 A1 bekannt. Dort wird eine Doppelkammerspritze mit unterschiedlichen Komponenten eines Klebstoffs in jeder Kammer, die einen gemeinsamen Druckkolben zum Austragen der Komponenten aus einer Austragöffnung der Spritze aufweisen, vorgesehen. Die Spritze ist durch einen Halter an einer Gasquelle angebracht oder mittels einer Leitung an eine Gasquelle angeschlossen, die Druckgas im Bereich der Austragöffnung der Spritze vorsieht. An dem Halter für die Spritze ist ferner ein Regulierungsventil vorgesehen, welches die Druckgasabgabe aus der Gasquelle reguliert. Eine Druckgasleitung ist von der Druckgasquelle zum Bereich der Austragöffnung für die beiden Komponenten an der Spritze geführt, so dass beim Austritt der Komponenten das Druckgas unmittelbar auf die Komponenten wirken kann. Bei der Anwendung dieser Austragvorrichtung wird zunächst mittels dem Regulierungsventil ein Gasdruck an der Austragöffnung erzeugt und anschliessend werden durch den Vorschub des Druckkolbens die Komponenten aus den Kammern der Doppelspritze ausgetragen. Diese gelangen unmittelbar nach dem Austritt in den Druckgasstrom und werden somit vernebelt und miteinander gemischt. Der Druckgasstrom lenkt das Gemisch anschliessend auf die Anwendungsoberfläche. Sofern das Sprühen unterbrochen wird oder die Spritze entleert ist, wird die Ausübung des Drucks auf den Druckkolben unterbrochen und der Gasstrom kann mittels dem Regulierungsventil abgestellt werden.

In der US 2006/0191962 A1 ist eine weitere Austragvorrichtung mit Druckgasunterstützung gezeigt, bei der die Austragvorrichtung in Form einer Doppelspritze mit einem Doppeldruckkolben an eine feststehende Druckgasquelle angeschlossen ist. Eine erste Leitung wird von der Druckgasquelle zu der Austragsöffnung der Spritze geführt, so dass aus der Öffnung austretende Komponenten vernebelt werden können. Eine zweite Leitung wird von der Druckgasquelle zu einem Kopfteil des Doppeldruckkolbens geführt, der in seinem Aufdrückbereich eine Öffnung aufweist, aus der ein Gasstrom, der durch die zweite Leitung geführt wird, austreten kann. Die Doppelspritze weist an ihrem Gehäuse mit den Spritzenkammern Gegendruckflächen auf, an welchen die Spritze mit den Fingern gehalten wird, während der Doppeldruckkolben mittels des Daumens in die Spritze hineingedrückt wird. Dabei wird die Öffnung im Druckbereich des Doppeldruckkolbens verschlossen und ein Austreten des Gasstroms wird verhindert. Dadurch wird in der Druckgasquelle ein Signal erzeugt, welches die Erzeugung eines Gasstroms in der ersten Leitung zur Austragöffnung der Austragvorrichtung auslöst, so dass die durch den Druck auf den Doppeldruckkolben ausgetragenen Komponenten durch den Gasstrom vernebelt werden.

Bei der Austragvorrichtung nach der US 2002/0165483 A1 sind nacheinander mehrere Handgriffe notwendig, um gleichzeitig das Austragen der Komponenten an der Austragvorrichtung und einen Gasstrom an der Austragöffnung der Austragvorrichtung zu erzeugen. Alternativ wäre es möglich, die Austragvorrichtung mit zwei Händen zu bedienen, um das Regulierungsventil und den Druckkolben gleichzeitig bedienen zu können. Bei der Austragvorrichtung nach US 2006/0191962 A1 sind wiederum zwei unterschiedliche, unabhängige Druckgasleitungen notwendig, um gleichzeitig mit dem Austragen der Komponenten aus der Doppelspritze einen Gasstrom an der Austragöffnung erzeugen zu können. Eine Regulierung des Gasstroms ist dabei nicht möglich.

US 5,582,596 offenbart in ihren Figuren 9-12 eine Austragvorrichtung, bei der eine Doppelspritze in einem pistolenartigen Applikator gehalten ist. Bei Betätigung eines Hebels, der nach Art eines Abzugs einer Pistole ausgestaltet ist, wird einerseits eine federbelastete Kolbenstange freigegeben, die die Kolben der Doppelspritze nach vorne drückt, um die Substanzen aus der Doppelspritze auszutragen. Andererseits wird durch die Betätigung des Hebels ein Druckgasventil geöffnet, um am Ausgang des Applikators einen Gasstrom zu erzeugen und dadurch die ausgetragenen Substanzen zu versprühen. Während diese Vorrichtung einfach zu bedienen ist, ermöglicht sie keine präzise Kontrolle über die ausgetragene Menge der Substanzen während des Sprühvorgangs. Ausserdem ist diese Vorrichtung verhältnismässig kompliziert aufgebaut und benötigt viel Platz.

Eine pistolenartige Austragvorrichtung zum Versprühen von Substanzen mit Druckgasunterstützung ist auch aus WO 95/31138 bekannt. Auch diese Vorrichtung benötigt viel Platz.

### DARSTELLUNG DER ERFINDUNG

Es ist eine Aufgabe der vorliegenden Erfindung, eine Austragvorrichtung zum Austragen eines Produkts mit Druckgasunterstützung vorzusehen, die einfach in der Handhabung ist, nur wenige Arbeitsschritte zum Austragen eines Produkts benötigt, einen geringen Druckgasverbrauch ermöglicht und eine kompakte Bauweise der Austragvorrichtung ermöglicht.

Diese Aufgabe wird durch eine Austragvorrichtung mit Druckgasunterstützung nach Anspruch 1 gelöst. Vorteilhafte Ausgestaltungen und weitere Ausführungsbeispiele gehen aus den Unteransprüchen hervor.
Eine Austragvorrichtung zum Austragen eines Produkts mit Druckgasunterstützung weist einen Behälter für das fluide Produkt auf, der eine Austragöffnung und ein Druckelement umfasst, das in dem Behälter verschieblich gelagert ist, um das Produkt aus dem Behälter durch die Austragöffnung austragen zu können. Der Behälter kann beispielsweise in Form einer herkömmlichen Spritze, insbesondere in Form einer Doppelkammerspritze, vorliegen. Das Produkt kann als wässrige Lösung oder auch als viskoses Produkt vorliegen. Ferner kann das Produkt aus mehreren, getrennten Komponenten bestehen, die beispielsweise jeweils in einer Kammer einer Doppelkammerspritze vorgesehen sind. Es ist möglich, die Komponenten des Produkts zunächst innerhalb des Behälters abzumischen und erst dann durch die Austragöffnung auszutragen. Bevorzugt werden jedoch unterschiedliche Komponenten durch Vernebelung mittels eines Druckgases ausserhalb der Austragvorrichtung vermischt. Als Druckelement kann beispielsweise ein Druckkolben dienen, der sich an einen Stopfen in dem Behälter, bzw. einen Stopfen in einer Komponentenkammer, anschliesst und diese im Behälter vorschieben kann, um so das Behältervolumen zu verkleinern und das Produkt durch die Austragöffnung auszudrücken. Vorzugsweise ist der Behälter länglich ausgebildet und weist an einem Ende die Austragöffnung auf und am gegenüberliegenden Ende kann das Druckelement in den Behälter eingedrückt werden.
Ferner ist an dem Behälter ein Gegendruckelement angeordnet, das bei einem Druck auf das Druckelement zum Vorschub des Druckelements in den Behälter hinein zur Aufnahme des dabei entstehenden Gegendrucks dient. Das Gegendruckelement ist fest mit dem Behälter verbunden oder kann abnehmbar an dem Behälter angebracht werden. Ist das Gegendruckelement an dem Behälter angebracht, ist es relativ zum Behälter feststehend. Zum Austragen des Produkts kann der Behälter z. B. mit den Fingern an dem Gegendruckelement ergriffen werden und das Druckelement kann mittels des Daumens in den Behälter hinein gedrückt werden, wobei der Behälter mit den Fingern am Gegendruckelement ortsfest gehalten werden kann. Das Gegendruckelement weist hierfür eine oder mehrere Gegendruckflächen auf, die vorzugsweise seitlich, bzw. an zwei sich gegenüberliegenden Seiten des Behälters vorgesehen sind.

Weiter weist die erfindungsgemässe Austragvorrichtung eine Druckgaseinrichtung zur Abgabe von Druckgas im Bereich der Austragöffnung und eine Betätigungseinrichtung zur Betätigung eines Ventils zur Steuerung der Druckgasabgabe von der Druckgaseinrichtung auf. Die Druckgaseinrichtung kann als herkömmliche Gaspatrone oder Spraydose in handlicher Form oder auch als ortsfester Druckgasgenerator vorgesehen sein. Das Druckgas kann mittels einer Zufuhrleitung zu dem Ventil und von dem Ventil mittels einer Abgabeleitung in den Bereich der Austragöffnung geführt werden. Im Bereich der Austragöffnung kann die Abgabeleitung an einen herkömmlichen Sprühkopf angeschlossen werden, der auf die Austragöffnung der Austragvorrichtung aufgesetzt werden kann, um eine geeignete Gasströmung um die Austragöffnung herum realisieren zu können. Derartige Sprühköpfe sind aus dem Stand der Technik bekannt. Die Austragöffnung zum Austragen des Produkts aus dem Behälter ist in diesem Fall durch die Austragöffnung des Sprühkopfes gegeben. Der Sprühkopf ist relativ zum Behälter fest angeordnet und kann somit theoretisch als Teil des Behälters betrachtet werden. Das Ventil kann mittels der Betätigungseinrichtung von einer geschlossenen Ventilposition, in der kein Gasstrom in die Abgabeleitung geführt wird, in eine zumindest teilweise geöffnete Ventilposition gebracht werden, in der ein Gasstrom von der Druckgaseinrichtung durch das Ventil in die Abgabeleitung und bis zur Austragöffnung geleitet wird.

Gemäss der vorliegenden Erfindung wird die Betätigungseinrichtung zur Betätigung des Ventils der Druckgaseinrichtung an dem Gegendruckelement vorgesehen, das an dem Behälter für das fluide Produkt angeordnet ist. Erfindungsgemäss kann daher gleichzeitig mit der Druckausübung, die zum Austragen des fluiden Produkts aus dem Behälter erforderlich ist, auch das Ventil der Druckgaseinrichtung geöffnet werden, womit ein Druckgasstrom im Bereich der Austragsöffnung des Behälters erzeugt wird. Die Erzeugung des Druckgasstroms und das Austragen des fluiden Produkts an der Austragsöffnung erfolgen daher durch denselben Handgriff. Es ist nicht erforderlich, in einem ersten Schritt durch eine Ventilöffnung den Gasstrom zu erzeugen und anschliessend in einem zweiten Schritt, während das Gas bereits ausströmt, das Produkt aus dem Behälter auszutragen. Dadurch kann der Verbrauch des Druckgases reduziert werden und die Handhabung der Austragvorrichtung wird vereinfacht. Die Steuerung des Druckgasstroms erfolgt innerhalb desselben Leitungsstrangs, der sich von der Druckgasquelle bis zur Austragöffnung am Behälter erstreckt. Die erfindungsgemässe Austragvorrichtung kommt daher mit wenigen Bauteilen aus und weist eine geringe Fehleranfälligkeit auf.
Nach einer Ausführungsform der Erfindung kann das Ventil als Absperrventil vorgesehen sein, das z. B. durch eine Ventilinnentrommel und eine Ventilaussentrommel gebildet wird, die konzentrisch zueinander angeordnet und zueinander drehbar sind. Die Druckgaszufuhrleitung kann in eine Öffnung in der Umfangswand der Ventilaussentrommel münden und somit das Druckgas in das innere der Trommel einleiten. Die Abgabeleitung kann an einem Ende aus der Ventilinnentrommel münden, wobei die Ventilinnentrommel in ihrer Umfangswand eine Öffnung aufweist, die mit der Öffnung der Aussentrommel in einer bestimmten Drehposition der Innen- und Aussentrommel zueinander in Deckung ist. Sofern die beiden Öffnungen übereinander zu liegen kommen, kann das Duckgas aus der Druckgasquelle durch die Zufuhrleitung und die beiden Öffnungen in die Ventilinnentrommel und von dort in die Abgabeleitung strömen. Befinden sich die beiden Trommeln in einer Drehposition, in der die beiden Öffnungen nicht in Deckung sind, wirkt die Aussenwand der Ventilinnentrommel als Absperrorgan des Absperrventils.

Die Betätigungseinrichtung am Gegendruckelement wird in Form wenigstens eines Hebels zur Betätigung des Ventils vorgesehen. Vorzugsweise steht die Drehachse des wenigstens einen Hebels im Wesentlichen senkrecht zur Vorschubrichtung des Druckelements und der Hebelarm des Hebels erstreckt sich vorzugsweise ebenfalls im Wesentlichen senkrecht zur Vorschubrichtung. Die Hebelfläche entlang der Längsseite des Hebels, d. h. die Fläche, die durch den Hebelarm gebildet wird, entspricht vorzugsweise der Gegendruckfläche, die am Gegendruckelement ausgebildet ist und auf die der Gegendruck beim Vorschub des Druckelements in den Behälter wirkt. Demnach wird gemäss der vorliegenden Erfindung in vorteilhafterweise gleichzeitig beim Ausüben eines Drucks auf Druck- und Gegendruckelement auch ein Druck auf die Hebelfläche, bzw. den Hebelarm, der Betätigungseinrichtung ausgeübt, wodurch der Hebel von einer Ruheposition in eine Betätigungsposition geschwenkt werden kann. Das Ventil wird dabei von einer geschlossenen Ventilposition, die einer Ruheposition der Austragvorrichtung entspricht, in eine zumindest teilweise geöffnete Ventilposition, die der Betätigungsposition der Austragvorrichtung entspricht, gebracht, so dass ein Gasstrom zur Austragsöffnung geführt wird. Vorzugsweise ist hierfür der Hebel unmittelbar mit der Ventilinnentrommel verbunden, so dass die Ventilinnentrommel gemeinsam mit dem Hebel betätigt wird und somit innerhalb der Ventilaussentrommel verdreht wird, sobald ein Druck auf das Gegendruckelement ausgeübt wird. In diesem Fall, kann die Ventilaussentrommel relativ zum Behälter in Ruhe bleiben. Alternativ wäre es natürlich möglich, den Hebel mit der Ventilaussentrommel zu verbinden und die Ventilinnentrommel feststehend zum Behälter anzuordnen. Bei dieser Ausführungsform entspricht somit die Achse der Ventilinnen- und Ventilaussentrommel der Drehachse des Hebels.

Bei einer Ausführungsform der Erfindung kann die Betätigungseinrichtung z. B. durch zwei Hebel ausgebildet sein, die parallel zueinander angeordnet sind und auf zwei gegenüberliegenden Seiten, z. B. links und rechts, des Behälters vorgesehen sind. Vorzugsweise weisen die beiden Hebel eine gemeinsame Drehachse auf.

Bei einer weiteren Ausführungsform ist es auch möglich, dass die Gegendruckfläche des Gegendruckelements nicht unmittelbar der Hebelfläche entlang des Hebelarms entspricht, sondern die Gegendruckfläche im Bereich der Drehachse des Hebels vorgesehen ist. Zum Austragen eines fluiden Produkts aus dem Behälter kann bei dieser Variante der Daumen am Druckelement angreifen und ein mittlerer Bereich eines Fingers kann an der Gegendruckfläche im Bereich der Drehachse des Hebels angreifen. Somit kann ein Druck zwischen dem Druckelement und dem Gegendruckelement aufgebaut werden, ohne dass unmittelbar eine Kraft auf den Hebel einwirkt. Der Hebel kann dann in dosierter Weise durch die Fingerkuppe des Fingers bewegt werden, während der mittlere Bereich der Finger den Gegendruck aufgrund des Vorschubs des Druckelements aufnimmt. Somit kann die Druckgaszufuhr an der Austragöffnung unabhängig vom Druck, der zum Austragen des fluiden Produkts notwendig ist, reguliert werden. Die Regulierung kann dabei dadurch erfolgen, dass die Öffnungen an der Ventilaussentrommel und der Ventilinnentrommel nur teilweise in Überlappung gebracht werden. Der Druckgasdurchfluss durch das Ventil ist dabei proportional zu einer mehr oder weniger grossen Überlappungsfläche der beiden Öffnungen.

In der beschriebenen Ausführungsform wird das Ventil der Druckgaseinrichtung als Drehventil, bzw. Eckventil, geschildert. Grundsätzlich ist es aber auch möglich für eine Austragvorrichtung nach der vorliegenden Erfindung andere Ventile beispielsweise ein Hubventil zu verwenden. In einem solchen Fall, muss die Betätigung des Ventils nicht durch eine Drehbewegung erfolgen, wie sie im vorherigen Beispiel durch einen Hebel ausgeführt wird. Es können andere Betätigungsmechanismen, wie z. B. Parallelbewegungen verwendet werden, die ebenfall mit dem Aufbau des Drucks zwischen Druckelement und Gegendruckelement erzeugt werden können. Vorzugsweise verläuft eine solche Parallelbewegung entgegen der Vorschubrichtung des Druckelements.

Die Betätigungseinrichtung kann mittels einer Feder derart vorgespannt sein, dass das Ventil in der Ruheposition der Betätigungseinrichtung in einer geschlossenen Position ist und bei Betätigung der Betätigungseinrichtung entgegen der Kraft der Feder in eine Betätigungsposition bewegt werden kann, in der es in eine wenigstens teilweise geöffnete Position gebracht ist. Durch die Vorspannung mittels der Feder wird sicher gestellt, dass das Ventil in der Ruheposition der Betätigungseinrichtung geschlossen ist und nur bei Betätigung der Austragvorrichtung durch eine Bewegung der Betätigungseinrichtung entgegen der Kraft der Feder in eine geöffnete Position gebracht wird. Die Feder ist beispielsweise als einfache Spiralfeder im Bereich der Drehachse eines Hebels vorgesehen, der als Betätigungseinrichtung dient.

Bei der vorliegenden Erfindung ist die Baugruppe, welche das Gegendruckelement, die Betätigungseinrichtung, das Ventil und optional auch die Zufuhr- und Abgabeleitung umfasst, mehrfach verwendbar ausgebildet. Die Baugruppe kann als Ganzes von einem Behälter abgenommen werden und auf einen neuen Behälter aufgesetzt werden. Hierfür kann z. B. eine Klipp- oder Rasteinrichtung vorgesehen sein, mit der die Baugruppe auf den Behälter aufgeschnappt werden kann. Dabei kommt beispielsweise ein Hebel der Betätigungseinrichtung seitlich an dem Behälter zu liegen, wobei der Hebelarm senkrecht zur Vorschubrichtung des Druckelements im Behälter verläuft. Am Behälter können Rastvorrichtungen oder Anschläge für die Halterung der Baugruppe, bzw. der Klipp- oder Rasteinrichtung, am Behälter vorgesehen sein, so dass die Baugruppe präzise relativ zum Behälter positioniert werden kann und nach dem Aufsetzen auf den Behälter relativ zu diesem unbeweglich gehalten wird. Die Abgabeleitung kann in einfacher Weise mit einem Sprühkopf verbunden werden, der auf die Austragöffnung des Behälters aufgesetzt ist. An die Zufuhrleitung kann beispielsweise mittels einer Luerverbindung ein Druckgasbehälter angeschlossen werden. Der Druckgasbehälter kann auf diese Weise ohne grossen Aufwand ausgetauscht werden.

Gemäss einer anderen Ausführungsform der vorliegenden Erfindung kann die Betätigungseinrichtung neben einer Gegendruckfläche eines gehäusefesten Gegendruckelements vorgesehen sein. Beispielsweise kann der Behälter derart gegriffen werden, das ein Daumen auf dem Druckelement zu liegen kommt und der Ring- und Mittelfinger auf je einer Gegendruckfläche des Gegendruckelements aufliegt. Mit dem Zeigefinger kann dann die neben der Gegendruckfläche angeordnete Betätigungseinrichtung unabhängig von einer Druckausübung auf das Druckelement des Behälters betätigt werden. Weist die Betätigungseinrichtung einen Hebel auf, kann dieser beweglich neben der gehäusefesten Gegendruckfläche angeordnet werden. Auch bei dieser Variante sind keine verschiedenen Handgriffe erforderlich, um zunächst einen Druckgasstrom aufzubauen und anschliessend das Produkt aus dem Behälter auszutragen. Das Austragen des Produkts und das Betätigen der Druckgaseinrichtung können aus der gleichen Halteposition der Austragvorrichtung heraus ausgeführt werden. Die Ausübung einer Kraft auf die Gegendruckfläche und die Betätigungseinrichtung kann jedoch unabhängig voneinander erfolgen.

Eine solche Vorrichtung kann nicht nur zum Aufsprühen von medizinischen Wundklebern oder Gewebeklebern eingesetzt werden, sondern auch in anderen, insbesondere nichtmedizinischen, Anwendungen. Die Erfindung kann somit zwar in therapeutischen oder chirurgischen Anwendungen eingesetzt werden, ist aber auf solche Anwendungen keineswegs beschränkt.

Bei einem Verfahren (Betriebsverfahren) zum Austragen eines Produkts aus einer Austragvorrichtung mit Druckgasunterstützung, wie sie oben beschrieben wurde, wird erfindungsgemäss die Austragvorrichtung mit einer Hand ergriffen, wobei der Daumen auf dem Druckelement und wenigstens ein Finger auf dem Gegendruckelement zu liegen kommt. Nach dem Ergreifen der Austragvorrichtung werden das Druckelement und das Gegendruckelement durch zusammendrücken des Daumens und des wenigstens einen Fingers zusammengedrückt. Dabei erfolgt gleichzeitig ein Vorschub des Druckelements in den Behälter und es kann eine Öffnung des Ventils durch eine Betätigung der Betätigungseinrichtung erfolgen. Die Betätigungseinrichtung kann dabei durch denselben Finger wie das Gegendruckelement oder auch durch einen weiteren Finger bedient werden.

### KURZE BESCHREIBUNG DER ZEICHNUNGEN

Bevorzugte Ausführungsformen der Erfindung werden im Folgenden anhand der Zeichnungen dargestellt, die lediglich zur Erläuterung dienen und nicht einschränkend auszulegen sind. Aus den Zeichnungen offenbar werdende Merkmale der Erfindung sollen einzeln und in jeder Kombination als zur Offenbarung der Erfindung gehörend betrachtet werden. In der Zeichnung zeigen:
- Fig. 1:: eine perspektivische Darstellung einer Austragvorrichtung gemäss der vorliegenden Erfindung,
- Fig. 2:: einen Längsschnitt durch eine Austragvorrichtung nach Figur 1,
- Fig. 3a:: eine schematische Darstellung einer Austragvorrichtung nach der Erfindung in einer Position mit geschlossenem Ventil,
- Fig. 3b:: einen Detaillängsschnitt durch ein Ventil der Austragvorrichtung nach Figur 3a,
- Fig. 4a:: eine schematische Darstellung einer Austragvorrichtung nach der Erfindung in einer Position mit geöffnetem Ventil,
- Fig. 4b:: einen Detaillängsschnitt durch ein Ventil der Austragvorrichtung nach Figur 4a,
- Fig. 5:: eine dreidimensionale Darstellung einer Baugruppe mit Gegendruckelement gemäss der vorliegenden Erfindung und
- Fig. 6:: eine Explosionsdarstellung der Baugruppe nach Figur 5.

### BESCHREIBUNG BEVORZUGTER AUSFÜHRUNGSFORMEN

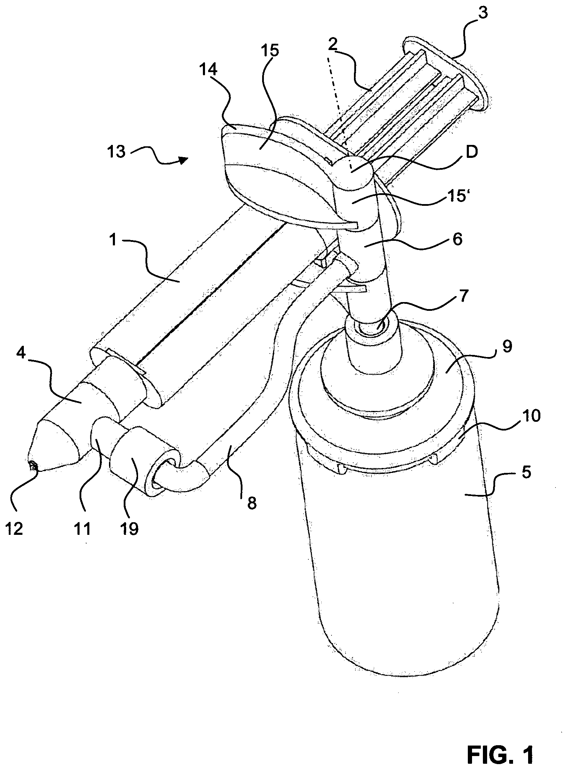

In Figur 1 ist eine Ausführungsform einer Austragvorrichtung mit Druckgasunterstützung gemäss der vorliegenden Erfindung gezeigt. Die Austragvorrichtung umfasst einen Behälter 1 in Form einer Doppelspritze mit zwei Kammern für unterschiedliche Komponenten eines Produkts. An einem Ende ist an der Doppelspritze ein Druckelement 2 vorgesehen, das als Doppelkolben ausgebildet ist, dessen beide Kolbenstangen jeweils in eine der Kammern der Doppelspritze ragen. Das Druckelement ist relativ zum Behälter, bzw. den Kammern, derart beweglich, dass es durch einen Druck auf eine Druckfläche 3 am Druckelement in die Kammern hineingeschoben werden kann, um das Volumen der Kammern zu verringern. Auf der dem Doppelkolben gegenüberliegenden Seite des Behälters ist eine Sprühdüse 4 über einer Austragöffnung des Behälters angeordnet. Die Sprühdüse 4 ist abnehmbar und kann beispielsweise mittels einer Bajonettverbindung am Behälter befestigt werden. Die Sprühdüse 4 weist eine oder auch mehrere Austragöffnungen 12 auf, durch die das Produkt, bzw. die Komponenten des Produkts aus der Austragvorrichtung ausgetragen werden.

Es ist eine Druckgaseinrichtung vorgesehen, die eine Druckgasquelle 5 in Form einer Gaspatrone, ein Ventil 6, eine Zufuhrleitung 7 zwischen der Druckgasquelle und dem Ventil und eine Abgabeleitung 8, die von dem Ventil 6 zur Sprühdüse 4 geführt ist, umfasst. Die Druckgasquelle 5 wird in einen Halter 9 eingesetzt, z. B. eingeschnappt, wobei eine Verbindung zwischen dem Druckgas und der Zufuhrleitung 7 hergestellt wird. Zur Befestigung der Druckgasquelle 5 am Halter 9 können z. B. Schnapparme 10 vorgesehen sein, die hinter einen Rand oder eine Kante an der Druckgasquelle eingreifen. Dabei wird ein Ventil an der Druckgasquelle 5 durch das Andrücken des Halters 9 aktiviert und somit die Druckgasverbindung hergestellt. Die Abgabeleitung 8 kann mittels einer Steckverbindung an einem Gaseinlass 11 der Sprühdüse 4 abnehmbar befestigt werden. Im vorliegenden Beispiel ist sie jedoch mit einer Überwurfmutter 19 an dem Gaseinlass 11 angebracht. Ein Druckgas aus der Gasquelle 5 kann somit über die Zufuhrleitung 7, das Ventil 6, die Abgabeleitung 8, den Gaseinlass 11 sowie interne Leitungen in der Sprühdüse 4 in den Bereich der Austragöffnung 12 geleitet werden.

An dem Ende des Behälters 1, aus dem das Druckelement 2 herausragt, ist ein Gegendruckelement 13 auf den Behälter aufgesteckt. Das Gegendruckelement 13 ist derart am Behälter befestigt, dass es in aufgestecktem Zustand relativ zum Behälter festsitzt. Die Steckverbindung ist jedoch lösbar ausgebildet, wie später erläutert wird. Das Gegendruckelement 13 dient dazu, eine Gegendruckfläche zu bieten, um den Gegendruck, der bei einem Druck auf das Druckelement zum Vorschub in den Behälter entsteht, aufnehmen zu können. Das Gegendruckelement 13 weist einen Hebel 14 mit einem Hebelarm auf, der um eine Drehachse D drehbar bzw. schwenkbar gelagert ist. Der Hebel 14 weist eine Gegendruckfläche 15 auf, die sich entlang des Hebelarms zwischen der Drehachse und dem gegenüberliegenden Endpunkt des Hebelarms erstreckt. Alternativ kann der Bereich des Hebels, der um die Drehachse angeordnet ist, als Gegendruckfläche 15' betrachtet werden. Der Hebel 14 ist um die Drehachse D schwenkbar und kann durch die Schwenkbewegung das Ventil 6 betätigen, das heisst das Ventil schliessen, teilweise öffnen oder vollständig öffnen. Der Hebel 14 wirkt somit als Betätigungseinrichtung am Gegendruckelement 13, mit der die Druckgaseinrichtung getätigt werden kann.
In der gezeigten Ausführungsform ist das Ventil 6, die Drehachse des Hebels 14 und die Zufuhrleitung 7 in einer Achse ausgerichtet. Grundsätzlich ist es aber auch möglich, eine Druckgasquelle zu verwenden, die stationär ist und mittels einer flexibel ausgebildeten langen Zufuhrleitung Druckgas zu dem Ventil führen kann. Die Drehachse D für das Ventil und den Hebel verläuft senkrecht zur Vorschubrichtung, in der das Druckelement 2 in die Kammern des Behälters 1 vorgeschoben wird. Die Gegendruckfläche 15 des Hebelarms ist annähernd senkrecht zur Vorschubrichtung und vorzugsweise senkrecht zur Drehachse ausgerichtet. Je nach Betätigungsstellung des Hebels 14 kann die Ausrichtung der Gegendruckfläche um diese senkrechte Stellung variieren.

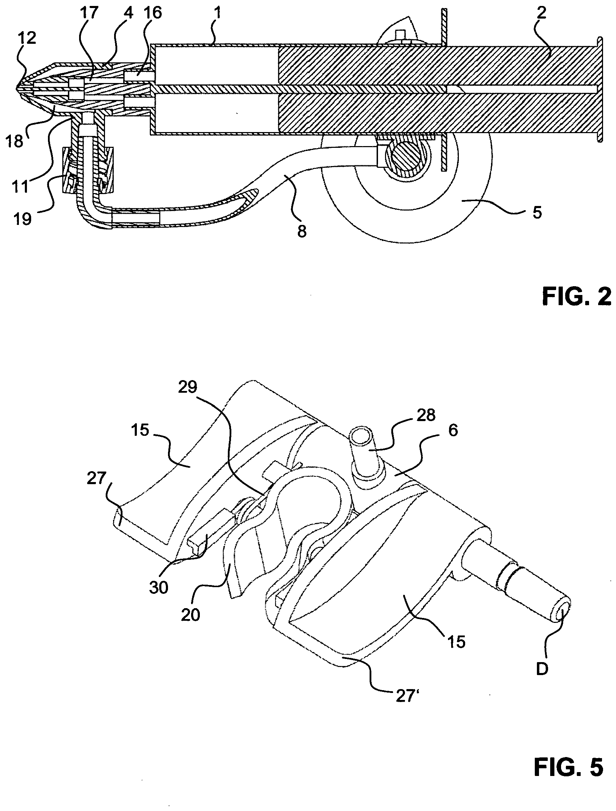

In Figur 2 ist ein Längsschnitt durch die Austragvorrichtung gemäss Figur 1 gezeigt. Das Druckelement 2 ist bis zur Hälfte der Kammervoluma des Behälters 1 in die Kammern eingeschoben. Das Druckelement 2 schliesst die Kammern stopfenartig dicht ab, so dass bei einem Vorschub des Druckelements 2 in den Behälter 1 die Komponenten in den Kammern durch Öffnungen 16 in die Sprühdüse 4 abgegeben werden können. Die Sprühdüse 4 weist Komponentenleitungen 17 und Druckgasleitungen 18 auf, die im Bereich der Austragöffnung 12 aus der Sprühdüse münden. Die Abgabeleitung 8 ist mittels der Überwurfmutter 19 an dem Gaseinlass 11 der Sprühdüse 4 befestigt.
Bei der erfindungsgemässen Betätigung der Austragvorrichtung werden gleichzeitig die Komponenten aus dem Behälter 1 durch die Komponentenleitungen 17 zur Austragöffnung 12 und ein Druckgasstrom der Druckgaseinrichtung durch die Abgabeleitung 8, den Gaseinlass 11 und die Druckgasleitungen 18 zur Austragöffnung 12 gefördert. Das an der Austragöffnung 12 austretende Druckgas kann die ebenfalls an der Austragöffnung 12 austretenden Komponenten vernebeln, so dass das gewünschte Klebstoffgemisch entsteht und das Gemisch kann mittels des Gasstroms auf eine Anwendungsfläche transportiert werden.

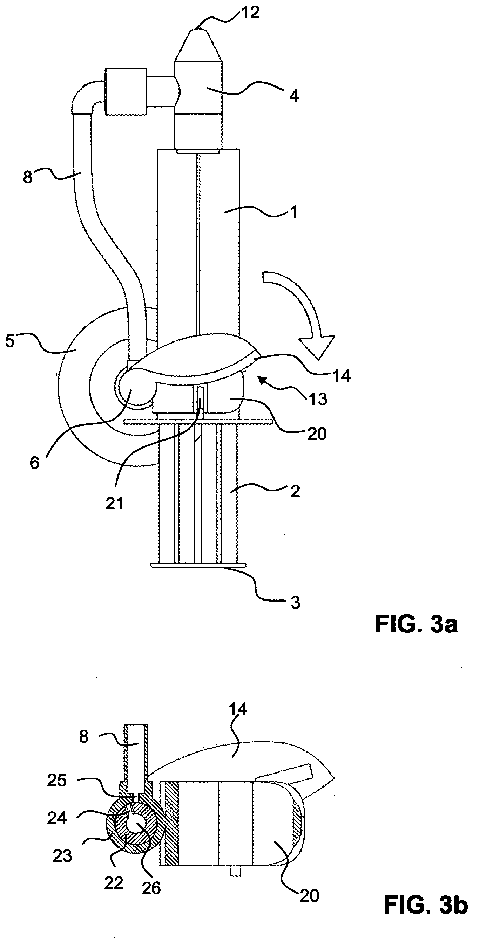

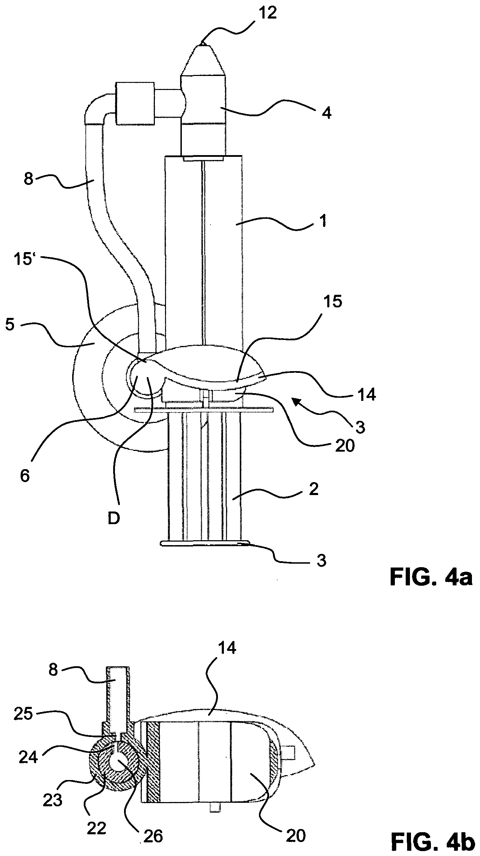

In Figur 3a ist die Austragvorrichtung in einer Ausgangsstellung, bzw. Ruhestellung, vor einem Austragen von Komponenten gezeigt. Das Ventil 6 befindet sich in einer geschlossenen Position, in der kein Druckgas in die Abgabeleitung 8 geleitet werden kann. Der Hebel 14 befindet sich in einer ersten Position, der Ruheposition. In dieser Position ist er von dem Druckelement 2, bzw. der Druckfläche 3 und dem Rand des Behälters, weg geschwenkt, d. h. er weist einen grösseren Abstand zum Endbereich des Behälters 1 auf, als in einer Betätigungsposition, die in Figur 4a beschrieben wird. Das Gegendruckelement 13 ist auf den Behälter 1 aufgesetzt, so dass die Drehachse des Hebels seitlich des Behälters und senkrecht zur Vorschubrichtung des Druckelements 2 zu liegen kommt. Zur Befestigung am Behälter 1 weist das Gegendruckelement eine Schnappklammer 20 auf, mit der das Gegendruckelement 13 auf dem Behälter befestigt werden kann. An dem Behälter 1 ist an der Aussenseite ein länglicher Vorsprung 21 vorgesehen, der als Distanzgeber zwischen der Schnappklammer 20 und dem Gehäuse 1 dient.

In Figur 3b ist ein Detailschnitt durch das Gegendruckelement 13 gezeigt, in dem das mit dem Hebel 14 gekoppelte Ventil 6 in einer Innenansicht sichtbar ist. Das Ventil ist als Absperrventil ausgebildet und umfasst eine Ventilinnentrommel 22 und eine Ventilaussentrommel 23. Die Trommeln 22 und 23 sind konzentrisch ineinander gelagert und gegeneinander drehbar. Die Ventilaussentrommel 23 ist fest an der Schnappklammer 20 des Gegendruckelements 13 angeordnet und ist somit relativ zum Behälter 1 in funktionsbereitem Zustand der Austragvorrichtung unbeweglich. Die Ventilinnentrommel 22 ist an den Hebel 14 gekoppelt, so dass durch Dreh- oder Schwenkbewegungen des Hebels 14, eine solche Bewegung auf die Ventilinnentrommel 22 übertragen wird. Die Ventilinnentrommel weist in ihrem Umfang eine Öffnung 24 und die Ventilaussentrommel weist in ihrem Umfang eine Öffnung 25 auf. In Figur 3b befindet sich das Ventil in einer geschlossenen Position, wobei die Innenumfangswand der Aussentrommel als Absperrorgan gegenüber der Öffnung 24 der Ventilinnentrommel wirkt. Im Inneren der Ventilinnentrommel 22 ist ein zylindrischer Hohlraum angeordnet, der als Gasstromleitung wirkt. Die Abgabeleitung 8 ist an diesen Hohlraum angeschlossen.

In Figur 4a ist die Austragvorrichtung in einer Betätigungsposition gezeigt, d. h. das Ventil der Druckgaseinrichtung ist zumindest teilweise geöffnet, so dass Druckgas aus der Druckgasquelle 5 durch das Ventil 6 zur Sprühdüse 4 strömen kann. Der Hebel 14 des Gegendruckelements 13 wurde in Richtung des Randes des Behälters 1 verschwenkt und weist somit einen geringeren Abstand zum Rand des Behälters 1 auf, als in der Ruheposition gemäss der Figur 3a.

Wie in Figur 4b ersichtlich ist, wurde dabei die Ventilinnentrommel 22 gemeinsam mit dem Hebel 14 innerhalb der Ventilaussentrommel 23 gedreht, so dass die Öffnung 24 der Ventilinnentrommel zumindest teilweise mit der Öffnung 25 der Ventilaussentrommel überlappt. Durch diese Überlappung wird eine Strömungsverbindung zwischen der Zufuhrleitung 7 der Druckgasquelle und der Abgabeleitung 8 zur Sprühdüse 4 hergestellt.

Zur Durchführung einer Austragung der Komponenten aus der Austragvorrichtung, bzw. eines Versprühens der Komponenten, ergreift ein Anwender die Austragvorrichtung mit einer Hand, beispielsweise von der linken Seite gemäss den Figuren 3a und 4a. Dabei kann der Behälter zwischen Zeige- und Mittelfinger zu liegen kommen. Die Druckfläche 3 des Druckelements 2 kann am Daumen zu liegen kommen. Der Zeige- und Mittelfinger kommen mit ihrem mittleren Bereich auf der Gegendruckfläche 15' in Bereich der Hebelachse D zu liegen, während die Fingerkuppen auf der Gegendruckfläche 15 entlang des Hebelarms aufliegen. Der Anwender kann nun die Finger und den Daumen zusammenpressen womit eine Kraft auf das Druckelement 2 zum Vorschub des Druckelements in den Behälter 1 und auf das Gegendruckelement 13, bzw. die Gegendruckfläche 15' ausgeübt werden. Während dem Zusammendrücken kann der Anwender durch gleichzeitiges Herabdrücken der Fingerkuppen den Hebel 14 betätigen, so dass das Ventil 6 in eine geöffnete Stellung gebracht wird. Somit kann gemäss der vorliegenden Erfindung gleichzeitig mit dem Vorschub des Druckelements 2 in den Behälter 1 zum Austragen der Komponenten aus dem Behälter 1 die Druckgaseinrichtung betätigt werden, so dass gleichzeitig mit dem Austragen der Komponenten ein Druckgasstrom aus der Austragöffnung austritt.

Alternativ ist es auch möglich, die Austragvorrichtung von der rechten Seite gemäss den Figuren 3a und 4a zu ergreifen, wobei ein Anwender die Austragvorrichtung unmittelbar zwischen den Fingerkuppen und dem Daumen hält. Bei dieser Vorgehensweise, wird die aufgewendete Kraft zum Vorschub des Druckelements 2 in Form des Gegendrucks unmittelbar auch auf die Gegendruckfläche 15 des Hebels 14 des Gegendruckelements 13 übertragen. Es ist daher nicht möglich, zu spät oder zu wenig Druckgas zum Versprühen der Komponenten aus der Austragvorrichtung auszulösen. Die Bewegung zum Austragen der Komponenten ist in dieser Ausführungsform untrennbar mit der Bewegung zur Betätigung des Ventils gekoppelt.

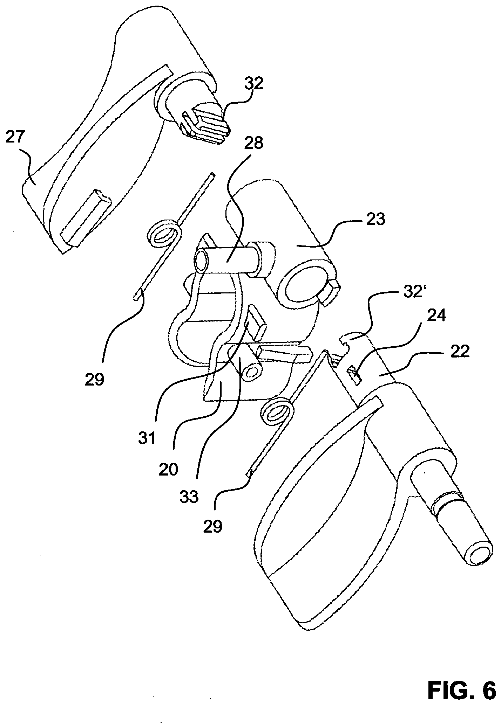

In den Figuren 5 und 6 ist eine Baugruppe des Gegendruckelements 13 gezeigt, die das Gegendruckelement 13, die Betätigungseinrichtung in Form des Hebels 14, das Ventil 6, die Zufuhr- und Abgabeleitungen 7 und 8, die Schnappklammer 20 und die Federn 29 umfasst. Die Schnappklammer 20 ist korrespondierend zur Form des Aussenumfangs des Behälters 3 ausgebildet, im vorliegenden Fall entsprechend der Aussenform einer Doppelspritze. Zu beiden Seiten neben der Schnappklammer 20 sind Hebelarme 27 des Hebels 14 gezeigt. Zwischen den Hebelarmen 27 ist das Ventil 6 mit einem Abgabestutzen 28 gezeigt. Die Hebelarme 27 und das Ventil 6 sind entlang der Drehachse des Hebels angeordnet. Zwischen den Hebelarmen 27 und der Schnappklammer 20 sind zu beiden Seiten Federn 29 vorgesehen, die zur Vorspannung des Hebels in eine Ruheposition dienen. Der Hebel 14 wird durch diese Federn in eine Ausgangsstellung der Austragvorrichtung gedrückt, in der das Ventil 6 geschlossen ist. Die Federn 29 stützen sich hierfür mit einem Ende an einer Schiene 30 an den Hebelarmen 27 und mit dem anderen Ende an einem Haltevorsprung 31 an der Schnappkammer 20 ab. Bei der Betätigung des Hebels werden die Hebelarme 27 durch Druck auf die Gegendruckflächen 15 relativ zu der Schnappklammer 20 verschwenkt, so dass die Federn 29 weiter gespannt werden.

Der erste Hebelarm 27 weist entlang der Achse D in Richtung des Ventils 6 eine Verbindungseinrichtung 32 auf mit der er mit einer Verbindungseinrichtung 32' an dem gegenüberliegenden zweiten Hebelarm 27 zusammenwirkt. Die Verbindungseinrichtungen 32 und 32' verbinden die Hebelarme fest miteinander. Die Verbindung kann z. B. durch eine Schnappverbindung realisiert werden.

An dem zweiten Hebelarm 27 ragt entlang der Drehachse D die Ventilinnentrommel 22 in Richtung des ersten Hebelarms 27 ab. Am Ende der Ventilinnentrommel 22 ist die Verbindungseinrichtung 32' vorgesehen. Die Ventilaussentrommel 23 ist als separates Bauteil gefertigt. Der Abgabestutzen 28 ragt über der Öffnung 23 der Ventilaussentrommel vom Aussenumfang der Ventilaussentrommel 23 ab. Zu beiden Seiten neben der Schnappklammer 20 sind die Federn 29 vorgesehen, die auf einem Federlager 33 an der Schnappklammer 20 gelagert sind und mit den in gegensätzliche Richtungen abragenden Federenden zwischen der Schiene 30 und den Haltevorsprüngen 31 zu liegen kommen. In zusammengesetztem Zustand sind die Verbindungseinrichtungen 32 und 32' und die Ventilinnentrommel 22 in die Ventilaussentrommel 23 eingesteckt und innerhalb der Ventilaussentrommel unlösbar miteinander verbunden, so dass in zusammengesetztem Zustand der Baugruppe die Ventilinnentrommel 22 innerhalb der Ventilaussentrommel 23 zu liegen kommt und die beiden Hebelarme 27 mittels der Federn 29 in einer vorgespannten Position relativ zur Schnappklammer 20 vorgespannt sind, in der die Öffnung 24 der Innentrommel und die Öffnung 25 der Aussentrommel sich nicht überlappen.

Die Baugruppe des Gegendruckelements 13 ist hier als eigenständige Baueinheit dargestellt, die auf einen Behälter aufgesetzt werden kann. Grundsätzlich ist es jedoch auch möglich anstatt der Schnappklammer 20 die einzelnen Bauelemente der Baugruppe unmittelbar an dem Behälter vorzusehen.

Die Erfindung wurde in den Figuren 1 bis 6 an einer Ausführungsform beschrieben. Grundsätzlich sind jedoch Varianten und Abwandlungen des Erfindungsgedanken möglich, die über die Darstellung der Zeichnungen hinausgeht. Beispielsweise kann anstelle des Drehventils ein Hubventil zur Betätigung der Druckgaseinrichtung vorgesehen werden, wie eingangs erwähnt wurde.

**BEZUGSZEICHENLISTE**

| | | | |
|---|---|---|---|
| 1 | Behälter, Doppelspritze | 17 | Komponentenleitung |
| 2 | Druckelement | 18 | Druckgasleitung |
| 3 | Druckfläche | 19 | Überwurfmutter |
| 4 | Sprühdüse | 20 | Schnappklammer |
| 5 | Druckgasquelle | 21 | Vorsprung |
| 6 | Ventil | 22 | Ventilinnentrommel |
| 7 | Zufuhrleitung | 23 | Ventilaussentrommel |
| 8 | Abgabeleitung | 24 | Öffnung (Innentrommel) |
| 9 | Halter | 25 | Öffnung (Aussentrommel) |
| 10 | Schnapparme | 26 | Hohlraum |
| 11 | Gaseinlass | 27 | Hebelarme |
| 12 | Austragöffnung | 28 | Abgabestutzen |
| 13 | Gegendruckelement | 29 | Feder |
| 14 | Hebel | 30 | Schiene |
| 15 | Gegendruckfläche | 31 | Haltevorsprung |
| 15' | Gegendruckfläche | 32, 32' | Verbindungseinrichtung |
| 16 | Öffnungen | 33 | Federlager |

## Patentansprüche

1. Austragvorrichtung zum Austragen eines Produkts mit Druckgasunterstützung, die dazu ausgebildet ist, im bestimmungsgemässen Gebrauch mittels einer Hand eines Benutzers gehalten und bedient zu werden, wobei die Austragvorrichtung aufweist:
einen Behälter (1) für das Produkt, mit einer Austragöffnung (12);
ein Druckelement (2), das in dem Behälter (1) verschieblich gelagert ist und dazu ausgebildet ist, im bestimmungsgemässen Gebrauch mit dem Daumen der genannten Hand in den Behälter hinein gedrückt zu werden;
ein Gegendruckelement (13), das an dem Behälter (1) angeordnet ist und bei einem Druck auf das Druckelement (2) zum Vorschub des Druckelements (2) in den Behälter zur Aufnahme eines dabei entstehenden Gegendrucks dient, wobei das Gegendruckelement (13) mindestens eine Gegendruckfläche (15') aufweist, die seitlich am Behälter angeordnet ist und dazu ausgebildet ist, den Behälter im bestimmungsgemässen Gebrauch mit mindestens einem Finger der genannten Hand ortsfest zu halten, während das Druckelement mit dem Daumen in den Behälter hinein gedrückt wird;
eine Druckgaseinrichtung (5, 7, 8) zur Abgabe von Druckgas im Bereich der Austragöffnung (12), mit einem Ventil (6) zur Steuerung der Druckgasabgabe; und
eine Betätigungseinrichtung (14) zur Betätigung des Ventils (6), wobei die Betätigungseinrichtung (14) derart an dem Gegendruckelement (13) angeordnet ist, dass die Betätigungseinrichtung im bestimmungsgemässen Gebrauch mit mindestens einem Finger der genannten Hand betätigbar ist, während gleichzeitig das Druckelement mit dem Daumen in den Behälter hinein gedrückt wird, **dadurch gekennzeichnet, dass** die Betätigungseinrichtung wenigstens einen Hebel (14) zur Betätigung des Ventils (6) umfasst, wobei der Hebel (14) eine Drehachse (D) aufweist, wobei die Gegendruckfläche (15') an einer Umfangsfläche des Hebels (14) im Bereich der Drehachse (D) ausgebildet ist, und wobei an dem Hebel (14) eine Betätigungsfläche zur Betätigung des Hebels (14) ausgebildet ist, so dass auf die Betätigungsfläche unabhängig vom Gegendruck auf die Gegendruckfläche (15') ein Betätigungsdruck ausgeübt werden kann.

2. Austragvorrichtung nach Anspruch 1, **dadurch gekennzeichnet, dass** das Gegendruckelement (13) vom Behälter (1) abnehmbar ist.

3. Austragvorrichtung nach Anspruch 1 oder 2, **dadurch gekennzeichnet, dass** die Betätigungseinrichtung (14) mittels wenigstens einer Feder (29) derart vorgespannt ist, dass sich das Ventil (6) in einer Ruheposition der Betätigungseinrichtung in einer geschlossenen Position befindet und dass das Ventil bei Betätigung der Betätigungseinrichtung in eine wenigstens teilweise geöffnete Position entgegen der Kraft der wenigstens einen Feder (29) beweglich ist.

4. Austragvorrichtung nach einem der vorhergehenden Ansprüche, **dadurch gekennzeichnet, dass** die Drehachse (D) des Hebels (14) im Wesentlichen senkrecht zur Vorschubrichtung des Druckelements (2) angeordnet ist.

5. Austragvorrichtung nach einem der vorhergehenden Ansprüche, **dadurch gekennzeichnet, dass** das Ventil (6) als Absperrventil mit einer Ventilinnentrommel (22) und einer Ventilaussentrommel (23) ausgebildet ist, die konzentrisch zueinander angeordnet und zueinander drehbar sind.

6. Austragvorrichtung nach Anspruch 5, **dadurch gekennzeichnet, dass** die Ventilinnentrommel (22) relativ zum Hebel (14) und die Ventilaussentrommel (23) relativ zum Behälter (1) feststeht.

7. Austragvorrichtung nach einem der Ansprüche 5 oder 6, **dadurch gekennzeichnet, dass** die Achse der Ventilinnentrommel (22) und der Ventilaussentrommel (23) der Drehachse (D) des Hebels (14) entspricht.

## Claims

1. A discharge apparatus for discharging a product and having compressed gas support, said discharge apparatus being designed so as, in its intended use, to be held and operated by means of one hand of a user, the discharge apparatus comprising:
a container (1) for the product, with a discharge opening (12);
a pressure element (2) which is mounted displaceably in the container (1) and is designed so as, in its intended use, to be pressed into the container by the thumb of said hand;
a counterpressure element (13) which is arranged on the container (1) and, when a pressure is applied to the pressure element (2) to advance the pressure element (2) into the container, serves to absorb a counterpressure arising in the process, wherein the counterpressure element (13) has at least one counterpressure surface (15') which is arranged laterally on the container and is designed so as, in its intended use, to hold the container in a fixed position with at least one finger of the abovementioned hand while the pressure element is pressed into the container by the thumb;
a compressed gas device (5, 7, 8) for dispensing compressed gas in the region of the discharge opening (12), with a valve (6) for controlling the dispensing of the compressed gas; and
an actuating device (14) for actuating the valve (6),
wherein the actuating device (14) is arranged on the counterpressure element (13) in such a manner that, in its intended use, the actuating device can be actuated with at least one finger of the abovementioned hand while at the same time the pressure element is pressed into the container by the thumb, **characterized in that** the actuating device comprises at least one lever (14) for actuating the valve (6), wherein the lever (14) comprises an axis of rotation (D), wherein the counterpressure surface (15') is formed on a circumferential surface of the lever (14) in the region of the axis of rotation (D), wherein an actuating surface for actuating the lever (14) is formed on the lever (14), so that it is possible for an actuating pressure to be exerted on the actuating surface independently of the counterpressure on the counterpressure surface (15').

2. The discharge apparatus as claimed in claim 1, **characterized in that** the counterpressure element (13) is removable from the container (1).

3. The discharge apparatus as claimed in claim 1 or 2, **characterized in that** the actuating device (14) is prestressed by means of at least one spring (29) in such a manner that the valve (6) is in a closed position in an inoperative position of the actuating device and, when the actuating device is actuated, the valve is movable into an at least partially open position counter to the force of the at least one spring (29).

4. The discharge apparatus as claimed in one of the preceding claims, **characterized in that** the axis of rotation (D) of the lever (14) is arranged substantially perpendicularly to the advancing direction of the pressure element (2).

5. The discharge apparatus as claimed in one of the preceding claims, **characterized in that** the valve (6) is designed as a shut-off valve with a valve inner drum (22) and a valve outer drum (23) which are arranged concentrically with respect to each other and are rotatable with respect to each other.

6. The discharge apparatus as claimed in claim 5, **characterized in that** the valve inner drum (22) is stationary relative to the lever (14) and the valve outer drum (23) is stationary relative to the container (1).

7. The discharge apparatus as claimed in either of claims 5 and 6, **characterized in that** the axis of the valve inner drum (22) and of the valve outer drum (23) corresponds to the axis of rotation (D) of the lever (14).

## Revendications

1. Un dispositif de distribution pour distribuer un produit sous assistance de gaz comprimé, formé pour être manié et tenu au moyen d'une main d'un utilisateur lors d'une utilisation conforme, où le dispositif de distribution comporte :
un récipient (1) pour le produit, muni d'une ouverture de distribution (12) ;
un élément de pression (2), qui est monté de manière coulissante dans le récipient (1) et qui est formé pour être poussé dans le récipient avec le pouce de la dite main lors d'une utilisation conforme ;
un élément de contre-pression (13), qui est disposé sur le récipient (1) et qui sert, lorsqu'une pression est exercée sur l'élément de pression (2) pour faire avancer l'élément de pression (2) dans le récipient, à absorber une contre-pression générée ce faisant, où l'élément de contre-pression (13) comporte au moins une surface de contre-pression (15') qui est disposée latéralement sur le récipient et qui est formée pour maintenir spatialement fixe le récipient avec au moins un doigt de la dite main lors d'une utilisation conforme, pendant que l'élément de pression est poussé dans le récipient avec le pouce ;
un dispositif de gaz comprimé (5, 7, 8) destiné à émettre du gaz comprimé dans la région de l'ouverture de distribution (12), muni d'une soupape (6) pour commander l'émission de gaz comprimé;
et un dispositif d'actionnement (14) pour actionner la soupape (6), où le dispositif d'actionnement (14) est disposé de telle sorte sur l'élément de contre-pression (13) que le dispositif d'actionnement peut être actionné avec au moins un doigt de la dite main lors d'une utilisation conforme, pendant que l'élément de pression est simultanément poussé dans le récipient avec le pouce, **caractérisé en ce que** le dispositif d'actionnement comprend au moins un levier (14) pour actionner la soupape (6), où le levier (14) comporte un axe de rotation (D), où la surface de contre-pression (15') est formée sur une surface circonférentielle du levier (14) dans une région de l'axe de rotation (D), et où une surface d'actionnement est formée sur le levier (14) pour actionner le levier (14), de sorte qu'une pression d'actionnement peut être exercée sur la surface d'actionnement indépendamment de la contre-pression sur la surface de contre-pression (15').

2. Un dispositif de distribution selon la revendication 1, **caractérisé en ce que** l'élément de contre-pression (13) est détachable du récipient (1).

3. Un dispositif de distribution selon la revendication 1 ou 2, **caractérisé en ce que** le dispositif d'actionnement (14) est précontraint de telle sorte au moyen d'au moins un ressort (29), que la soupape (6) se trouve dans une position fermée dans une position de repos du dispositif d'actionnement et que lors de l'actionnement du dispositif d'actionnement la soupape peut être déplacée vers une position au moins partiellement ouverte encontre de la force de l'au moins un ressort (29).

4. Un dispositif de distribution selon une des revendications précédentes, **caractérisé en ce que** l'axe de rotation (D) du levier (14) est disposé sensiblement de manière perpendiculaire par rapport à la direction d'avance de l'élément de pression (2).

5. Un dispositif de distribution selon une des revendications précédentes, **caractérisé en ce que** la soupape (6) est formée en tant que soupape de verrouillage munie d'un tambour interne de soupape (22) et d'un tambour externe de soupape (23), qui sont disposés de manière concentrique l'un par rapport à l'autre et sont rotatifs l'un par rapport à l'autre.

6. Un dispositif de distribution selon la revendication 5, **caractérisé en ce que** le tambour interne de soupape (22) est fixe par rapport au levier (14) et le tambour externe de soupape (23) est fixe par rapport au récipient (1).

7. Un dispositif de distribution selon une des revendications 5 ou 6, **caractérisé en ce que** l'axe du tambour interne de soupape (22) et du tambour externe de soupape (23) correspond à l'axe de rotation (D) du levier (14).
